# FASCICULE DE BREVET EUROPEEN

(11) **EP 0 593 657 B1**
(45) Date de publication et mention de la délivrance du brevet: **22.11.1995**
(21) Numéro de dépôt: 92915728.7
(22) Date de dépôt: 06.07.1992
(51) Int. Cl.: A61K 7/38, A61K 7/035, A61K 7/04, A61K 7/32, A61K 7/48

(54) **COMPOSITION DEODORANTE CORPORELLE EN POUDRE**
PULVERFOERMIGES KOERPERDEODORANT
POWDERED BODY DEODORANT COMPOSITION

(30) Priorité: 11.07.1991 FR 9108725
(43) Date de publication de la demande: 27.04.1994
(73) Titulaire: MALKA, Daniel, F-91230 Montgeron (FR)
(72) Inventeur: MALKA, Daniel, F-91230 Montgeron (FR)
(74) Mandataire: Lordonnois, Michel
(86) Numéro de dépôt international: FR9200640
(87) Numéro de publication internationale: WO9300885

(56) Documents cités:
- DE-A- 1 956 818
- US-A- 2 145 583
- CHEMICAL ABSTRACTS, vol. 115, no. 21, 25 Novembre 1991, Columbus, Ohio, US; abstract no. 239365c, page 469 ;

## Description

La présente invention concerne une composition déodorante corporelle en poudre, plus particulièrement conçue à base d'alun officinal et de gomme arabique, pour supprimer la croissance des bactéries donnant naissance à des mauvaises odeurs dues à l'excès de sudation. Cette composition déodorante, sous forme de cosmétique en poudre, est notamment étudiée pour que, suite à des applications sur l'épiderme, l'excès de sudation soit amplement réduit et que les mauvaises odeurs soient éliminées par suite de la suppression de la formation de bactéries.

Dans la technique antérieure, on connaît une crème déodorante anti-sudation contenant du sulfate d'alumine, de la gomme soluble dans l'eau, un sel de zinc également soluble dans l'eau et de la poudre inerte, cette crème étant décrite dans le brevet US-A-2.145.583.

On connaît également une poudre déodorante contenant du sulfate d'alumine et de potassium définie dans l'abrégé publié dans CHEMICAL ABSTRACTS - vol.115, n° 21, du 25 novembre 1991.

Par rapport à ces produits connus et bien qu'il contienne, comme eux, de l'alun à titre de composant actif, c'est-à-dire utilisé pour ses propriétés astringentes, le mélange en poudre selon la présente invention n'est pas conçu pour être utilisé, comme la crème qui précède, en tant que produit applicable après rasage, ni comme la poudre déodorante définie dans l'abrégé cité ci-dessus, en tant que produit d'entretien interne de chaussures. En effet, le but de la présente invention est de proposer un mélange en poudre qui réponde au traitement énoncé dans le préambule et contenant en quantités égales pour fournir 70% de sa quantité totale, de l'alun officinal sous forme de sulfate double d'alumine et de potassium et de la gomme arabique extraite de l'acacia, ainsi qu'une quantité d'oxyde de zinc de 5% et la quantité restante en poudre inerte.

Après avoir fait différents mélanges entre la poudre d'alun officinal et la poudre de gomme arabique, tout en incorporant dans ces mélanges 5% d'oxyde de zinc et 25% de poudre inerte, l'inventeur s'est aperçu que le mélange de poudres le plus satisfaisant était celui qui contenait spécifiquement en quantités: 35% d'alun officinal et 35% de gomme arabique.

## Revendications

1. Composition déodorante corporelle en poudre, applicable sur l'épiderme et contenant de l'alun en tant que composant actif, caractérisé par le fait que l'alun est l'alun officinal constitué par du sulfate double d'alumine et de potassium présent dans le mélange avec de la gomme arabique extraite de l'acacia, ces deux ingrédients étant présents en quantité sensiblement égale et composant, à eux deux, 70% de la quantité totale du mélange, lequel contient, en outre, de l'oxyde de zinc suivant une quantité de 5% et la quantité restante de la quantité totale du mélange étant sous forme de poudre inerte.

2. Composition déodorante corporelle en poudre selon la revendication 1, caractérisée par le fait qu'elle est constituée en quantités de 35% d'alun officinal, de 35% de gomme arabique, de 5% d'oxyde de zinc et de 25% de poudre inerte.

## Claims

1. A powdered body deodorant composition to be applied to the skin and containing alum as active ingredient, characterized in that the alum is officinal alum consisting of double aluminium potassium sulphate mixed with gum arabic extracted from acacia, these two ingredients being present in substantially equal quantities and constituting between them 70 % of the total quantity of the mixture, which further contains zinc oxide in an amount of 5 % and the remaining quantity of the total quantity of the mixture being in the form of inert powder.

2. A powdered body deodorant composition according to claim 1, characterized in that it consists of officinal alum in the amount of 35 %, gum arabic in the amount of 35 %, zinc oxide in the amount of 5 % and inert powder in the amount of 25 %.

## Patentansprüche

1. Pulverförmiges Körperdeodorant, das auf der Haut anwendbar ist und Alaun als aktive Komponente enthält, dadurch gekennzeichnet, daß das Alaun das offizinelle, aus dem Doppelsulfat aus Aluminium und Kalium bestehende Alaun ist, das in dem Gemisch mit aus der Akazie extrahiertem Gummi arabicum vorliegt, wobei diese beiden Ingredienzien in einer im wesentlichen gleichen Menge vorliegen und miteinander 70 % der Gesamtmenge des Gemischs bilden, das außerdem 5% Zinkoxid enthält, und wobei die restliche Menge der Gesamtmenge des Gemischs in Form von inertem Pulver vorliegt.

2. Pulverförmiges Körperdeodorant nach Anspruch 1, dadurch gekennzeichnet, daß es zu 35 % aus offizinellem Alaun, zu 35 % aus Gummi arabicum, zu 5 % aus Zinkoxid und zu 25 % aus inertem Pulver besteht.
